Europäisches Patentamt

European Patent Office

Office européen des brevets

(19)

(11) Veröffentlichungsnummer: **0 268 680**
A1

(12) **EUROPÄISCHE PATENTANMELDUNG**

Veröffentlicht nach Art. 158 Abs. 3 EPÜ

(21) Anmeldenummer: 86904987.4

(22) Anmeldetag: 29.05.86

Daten der zugrundeliegenden internationalen Anmeldung:

(86) Internationale Anmeldenummer:
PCT/SU86/00052

(87) Internationale Veröffentlichungsnummer:
WO87/07154 (03.12.87 87/27)

(51) Int.Cl.³: **A 61 L 15/03**

(43) Veröffentlichungstag der Anmeldung:
01.06.88 Patentblatt 88/22

(84) Benannte Vertragsstaaten:
BE CH DE GB IT LI SE

(71) Anmelder: VSESOJUZNY
NAUCHNO-ISSLEDOVATELSKY I ISPYTATELNY
INSTITUT MEDITSINSKOI TEKHNIKI
ul. Kasatkina, 3
Moscow, 129301(SU)

(71) Anmelder: MOSKOVSKY
NAUCHNO-ISSLEDOVATELSKY ONKOLOGICHESKY
INSTITUT IMENI P.A. GERTSENA
2-oi Botkinsky proezd, 3
Moscow, 125284(SU)

(72) Erfinder: BELYKH, Sergei Ivanovich
2-oi Krestovsky per., 4-66
Moscow, 129041(SU)

(72) Erfinder: DAVYDOV, Anatoly Borisovich
ul. Krasny Kazanets, 19-1-283
Moscow, 111395(SU)

(72) Erfinder: ILIINA, Anna Ivanovna
Moskovsky pr., 3-17 Moskovskaya obl.
Pushkino, 141200(SU)

(72) Erfinder: KESHELAVA, Viktor Vladimirovich
Zvenigorodskoe shosse, 17-4-68
Moscow, 109387(SU)

(72) Erfinder: OLSHANSKY, Vladimir Olegovich
ul. 13 Parkovaya, 27-4-45
Moscow, 105215(SU)

(72) Erfinder: FIRSOVA, Elizaveta Vasilievna
ul. Ostrovityanova, 27-2-93
Moscow, 117437(SU)

(72) Erfinder: SCHITKOV, Kirill Georgievich
ul. 15 Parkovaya, 46-4-41
Moscow, 105523(SU)

(74) Vertreter: von Füner, Alexander, Dr. et al,
Patentanwälte v. Füner, Ebbinghaus, Finck Mariahilfplatz
2 & 3
D-8000 München 90(DE)

(54) **ANTITUMORFILM AUS BIOLOGISCH ABBAUBAREN POLYMEREN.**

(57) Der geschwulsthemmende Film aus biodestruktierbaren Polymeren stellt einen zweischichtigen Film dar, in welchem die erste an die Geschwulstgewebe anliegende Schicht aus einem biodestruktierbaren Polymer und einem Zytostatikum und die zweite Schicht aus demselben oder einem anderen biodestruktierbaren Polymer besteht, das eine längere Biodestruktionsdauer als die Iliminierungsdauer des Zytostatikums aus der ersten Schicht hat, dabei beträgt die Dicke der zweiten Schicht 0, 1 bis 0,8 Teile von der ersten Schicht.

GESCHWULSTHEMMENDER FILM AUS BIODESTRUKTIERBAREN POLYMEREN

## Gebiet der Technik

Die vorliegende Erfindung bezieht sich auf die Medizin und betrifft insbesondere einen geschwulsthemmenden Film aus biodestruktierbaren Polymeren.

## Voranstehender Stand der Technik

Es ist die intravenöse Einführung der Zytostatika bekannt (N.I. Dyatlova, A.I. Starshinin "Prospidin v kompleksnom lechenii raka legkogo" "Prospidin in der komplexen Behandlung des Lungentumors", Sammlung der Arbeiten von "S.Ordzhonikidze" VNIKhFI. "Prospidin - ein neues Zytostatikum", H. 3, Moskau, 1973, S. 190-195). Die Gesamtdosierung beträgt 2,5 bis 3,5 g für die Behandlungskur. Solch eine Methode wird von mehreren Komplikationen begleitet. Bei 30 % der Kranken werden nach 2 bis 3 Injektionen Schwindel, Kopfschmerzen, Appetitverlust, Erbrechen nachgewiesen. Bei einer Reihe der Kranken entwickeln sich zu Ende der Behandlungskur Parästhesien in der Gegend der Finger und Zungenspitze.

Es ist auch die Einführung der Zytostatika durch Infiltration der Gewebe mit der Prospidinlösung in der 0,5%igen Novokainlösung bekannt (T.A. Kunitsina "Primenenie prospidina v khirurgicheskoi klinike" "Anwendung von Prospidin in der chirurgischen Klinik", Sammlung der Arbeiten von "S.Ordzhonikidze" VNIKhFI "Prospidin - ein neues Zytostatikum", H. 3, Moskau, 1973, S. 204-208). Diese Methode hat auch ihre Nachteile. An Stelle der Infiltration entwickelt sich die Nekrose und der Prozeß der Wundheilung verzögert sich stark. Die Wunden heilen durch die sekundäre Spannung frühestens 2 Monate nach der Abtrennung der nekrotischen Gewebe aus.

Es ist die Verwendung der Filmstoffe aus Kollagen, die physiologisch wirksame Präparate, beispielsweise antimikrobielle Arzneimittel enthalten, bei chirurgischen Eingriffen in der Ophthalmologie bekannt ("Farmatsiya", Moskau, 1979, H. 4, S. 31-34).

Jedoch ist das Anwendungsgebiet für Filmstoffe aus Kollagen begrenzt. Die Verwendung dieser Filmstoffe in der Onkologie ist unmöglich, weil die Implantation dieser Filme (ein Polymer tierischer Herkunft) entzündliche Reaktionen der anliegenden Gewebe vom immunen Charakter hervorruft. Solche Reaktionen sind in der Zone der bösartigen Geschwulste besonders gefährlich. Außerdem können solche Filme wegen einer hohen Hydrophilie und einer geringen Biodestruktionszeit keine langwierige Eliminierung des Arzneimittels bewirken, die für die Wachstumshemmung der nach dem operativen Eingriff gebliebenen Geschwulstzellen notwendig ist.

Offenbarung der Erfindung

Der erfindungsgemäße geschwulsthemmende Film aus biodestruktierbaren Polymeren ist neu und wurde in der Fachliteratur nicht beschrieben.

Der Erfindung liegt die Aufgabe zugrunde, einen neuen geschwulsthemmenden Film aus biodestruktierbaren Polymeren zu entwickeln, der eine allmähliche langwierige Eliminierung des Arzneimittels in die Einführungszone bewirkt.

Die Aufgabe wird dadurch gelöst, daß der geschwulsthemmende Film aus biodestruktierbaren Polymeren erfindungsgemäß einen zweischichtigen Film darstellt, in welchem die erste an die Geschwulstgewebe anliegende Schicht aus einem biodestruktierbaren Polymer und einem Zytostatikum besteht, und die zweite Schicht aus demselben oder einem anderen biodestruktierbaren Polymer besteht, bei dem die Biodestruktionszeit höher als die Zeit der Eliminierung des Zytostatikums aus der ersten Schicht ist, wobei die Dicke der zweiten Schicht von 0,1 bis 0,8 Teile von der Dicke der ersten Schicht beträgt. Der erfindungsgemäße Film enthält als biodestruktierbares Polymer der ersten Schicht vorzugsweise ein Polymer auf Grundlage von Zyanakrylaten oder ein Kopolymer aus N-Vinylpyrrolidon mit Alkylakrylat und/oder Alkylmethakrylat. Als biodestruktierbares Polymer der zweiten Schicht enthält der erfindungsgemäße Film vorzugsweise ein Polymer auf Grundlage von Zyanakrylaten, Polyglykolid, Karboxymethylzellulose oder ein Kopolymer aus N-Vinylpyrrolidon mit Akrykakrylat und/oder Alkylmethakrylat.

Der erfindungsgemäße Film gestattet, die therapeutische Einwirkungsdauer des Arzneimittels im Vergleich zur Injektionseinführung des Arzneimittels um das 6 - 30fache zu erhöhen, die Dauer der Aufrechterhaltung der therapeutischen

Konzentration in der Geschwulstzone um das 6 .— 30fache zu erhöhen, den Verbrauch an Zytostatika um das 3-30fache zu reduzieren.

Die beste Ausführungsform der Erfindung

Als geschwulsthemmende Arzneimittel kann der erfindungsgemäße geschwulsthemmende Film Bis-(β-chloräthyl)aminderivate, Derivate, die Äthylenamingruppen enthalten, Zytostatika, Antimetabolite, geschwulsthemmende Antibiotika, Naturalkaloide enthalten.

Die Bereitstellung des erfindungsgemäßen Films wird durch Gießen aus der Lösung mit darauffolgender Trocknung bei Zimmertemperatur oder bei einer höheren Temperatur durchgeführt, die die Wirksamkeit des Arzneimittels nicht beeinträchtigt. Das Auftragen der zweiten Schicht, die die Schutzfunktion erfüllt, wird nach der Trocknung der ersten Schicht verwirklicht.

Die Verwendung des erfindungsgemäßen Films mit einer Dicke der zweiten Schicht von weniger als 0,I Teil von der Dicke der ersten Schicht schafft technologische Schwierigkeiten bei deren Auftragen und sichert keine zuverlässige Vergrößerung der Eliminierungsdauer des Arzneimittels.

Die Verwendung des erfindungsgemäßen Films mit einer Dicke der zweiten Schicht von mehr als 0,8 Teile von der Dicke der ersten Schicht ruft keine weitere Erhöhung der Eliminierungsdauer hervor, sondern vergrößert nur den Anteil des unwirksamen Teils des Arzneimittels.

Der erfindungsgemäße Film wurde im Tierversuch und in Kliniken an Menschen geprüft.

Im Tierversuch wurden folgende geschwulsthemmende Filme verwendet.

I. Ein geschwulsthemmender Film, dessen erste Schicht aus dem Kopolymer von N-Vinylpyrrolidon und Butylmethakrylat und/N,N''-Di( γ-chlor-ß-oxypropyl)-N,N'-dispirotriperazonium/-dihydrochlorid (Prospidin) und die zweite Schicht aus demselben Kopolymer besteht.

2. Ein geschwulsthemmender Film, dessen erste Schicht aus dem Kopolymer von N-Vinylpyrrolidon und Butylmethakrylat und /N,N''-Di( γ-chlor-ß-oxypropyl)-N,N'-dispirotripera-

zonium/-dihydrochlorid und die zweite Schicht aus einem Gemisch von Polyäthylzyanakrylat mit Polyäthoxyzyanakrylat in einem Verhältnis von I:I besteht. Der Gehalt am genannten Zytostatikum Prospidin in den Mustern betrug 20 Gew.%, d.h. 22,5 mg.

Die zweite Schicht, deren Dicke 0,3 bis 0,5 der Dicke der ersten Schicht beträgt, wird auf die äußere der Wundoberfläche gegenüberliegende Filmschicht aufgetragen, um die vorteilhafte Eliminierung des Arzneimittels in Richtung der Anlagerung der Geschwulstzellen zu sichern sowie darin eine höhere Konzentration zu schaffen und zu sichern.

Die Muster wurden in 2-3 hermetische Polyäthylenpakete verpackt und durch ɣ-Bestrahlung in Dosen sterilisiert, die 2,75 mRad nicht übersteigen.

Die Prüfungen verfolgten das Ziel, funktionelle Anwendbarkeit der entwickelten Muster (deren Effektivität gegen das Entstehen der postoperativen Rezidive) festzustellen und die mögliche Indikationen zur Anwendung vorläufig zu bestimmen.

Die Untersuchungen wurden an 80 geschlechtsreifen männlichen Ratten der Wistar-Gattung (von 200 bis 240 g Körpergewicht) mit einem unter die Haut der vorderen Bauchfellwand transplantierten Karzinom PC-I durchgeführt. Diese Geschwulst rezidiviert nach der chirurgischen Entfernung mindestens in 90 % der Fälle. I4 bis I7 Tage nach der Transplantation bei einem Geschwulstausmaß von I,5 bis 2,5 cm wurden diese Geschwulste unter Zurücklassen eines geringen Fragments des Geschwulstgewebes (0,2 x 0,3 - 0,4 mm) ausgeschnitten. Die Operation wurde unter Äthernarkose erfüllt. Auf die Wundoberfläche wurden die Prüfmuster des erfindungsgemäßen Films so implantiert, daß sich das zurückgebliebene Fragment des Geschwulstgewebes ungefähr im Filmzentrum befand, die Haut über dem Film wurde mit seidenen Knotennähten zugenäht, oben wurde eine sterile Mullrolle aufgetragen. In Kontrollgruppen wurden die Tiere nach der Entfernung der Geschwulst keinen zusätzlichen Manipulationen ausgesetzt oder sie erhielten Prospidin intramuskulär innerhalb von 8 Tagen in einer Menge von 60 mg pro I kg Körpergewicht (die Gesamtdosis des Präparats betrug ungefähr 96 mg). Die Effektivität der Implan-

tationsmethode des erfindungsgemäßen Films mit Prospidin wurde nach der Dauer der Remissionsperiode, der Häufigkeit des Auftretens der Rezidive sowie nach der Überlebenszeit der Versuchstiere im Vergleich zur Kontrolle eingeschätzt, d.h. zu den Tieren, denen keine Filmimplantation durchgeführt wurde, oder zu den Tieren, die das Zytostatikum in freier Form erhielten.

Die Beobachtungen zeigten, daß die Tiere die Implantation des erfindungsgemäßen Films mit Prospidin gut vertragen. Es wurden keine Äußerungen der toxischen Wirkung des im Film enthaltenden Prospidins nachgewiesen, einschließlich Veränderungen, die von der Entwicklung einer Immunodepression (Herabsetzung des Gewichts von Thymus, Lymphknoten u.a.) zeugen. Bei der Kurbehandlung mit dem Präparat wurden in der Kontrolle die Merkmale des toxischen Einflusses auf den Organismus verzeichnet. Es wurde kein Tod der Tiere beobachtet, der auf die Anwendung des erfindungsgemäßen Films zurückzuführen war. Im Ergebnis der durchgeführten Untersuchungen wurde die funktionelle Tauglichkeit des erfindungsgemäßen Films festgestellt. Bei allen operierten Tieren sowohl in der Kontrolle als auch im Versuch entwickelten sich zu verschiedenen Zeiten Rezidive. Jedoch stieg bei Tieren mit dem implantierten Film die Dauer der Remissionsperiode im Vergleich zur Kontrolle ungefähr um das 2,0 - 2,5fache an (von 9 Tagen in der Kontrolle auf 18 bis 24 Tage). Die Tempi des Geschwulstwachstums bei den Versuchstieren in allen Gruppen, die den erfindungsgemäßen Film erhielten, waren bedeutend herabgesetzt. So war das Geschwulstvolumen bei diesen Tieren zum 35. Beobachtungstag ungefähr um 7mal geringer als in der Kontrolle.

In Versuchen mit der Implantation des erfindungsgemäßen Films auf die Wundoberfläche betrug die Überlebenszeit der Tiere 98 bzw. 120 Tage (in der Kontrollgruppe - 31 Tage). Bei der 8maligen intramuskulären Einführung von Prospidin überstieg die Gesamtdosis von Prospidin die im erfindungsgemäßen Film mindestens um das 4fache, die Überlebenszeit erhöhte sich im Vergleich zur Kontrolle auf 90 Tage, was im Vergleich zur Verwendung des erfindungsgemäßen Films auch weniger ist.

Somit legten die Ergebnisse der durchgeführten Prüfun-

- 6 -

gen die Möglichkeit an den Tag, den erfindungsgemäßen geschwulsthemmenden Film zur Vorbeugung der Entwicklung der postoperativen Rezidive effektiv anzuwenden. Das Ausbleiben der toxischen Wirkung von Prospidin in der filmförmigen Arzneiform, das mit der dosierten Eliminierung des Präparats aus dem Film verbunden ist, und einen deutlich ausgeprägten erhöhten geschwulsthemmenden Effekt zeigten deutlich die Vorteile der Verwendung des erfindungsgemäßen Films vor anderen bekannten Arzneimitteln.

Die klinischen Prüfungen der erfindungsgemäßen Filme wurden an Filmmustern mit einem Prospidingehalt von 2 Gew.% und 20 Gew.% durchgeführt.

Die Filme auf Grundlage eines Kopolymers aus N-Vinylpyrrolidon und Butylmethakrylat in der ersten und zweiten Schicht mit einem Prospidingehalt von 2 Gew.% wurden auf das Bett der Gehirngeschwulst nach deren subtotalen Exstirpation appliziert. Solch eine Applikation führt zur Erhöhung der Überlebensdauer um 25 bis 30 %. Ähnliche Filme mit einem Prospidingehalt von 20 Gew.% wurden in weiche Gewebe nach der Entfernung des Kehlkopf- und Mammakarzinoms (21 Patienten) implantiert. Die klinischen Prüfungen zeigten die Verminderung der Anzahl der Geschwulstrezidive im Vergleich zur Kontrollgruppe um 38 %.

Zum besseren Verständnis der vorliegenden Erfindung werden konkrete Beispiele des erfindungsgemäßen geschwulsthemmenden Films angeführt.

Beispiel I.

Ein geschwulsthemmender Film, dessen erste Schicht aus 98 Gew.% Kopolymer von N-Vinylpyrrolidon und Butylmethakrylat und 2 Gew.% Prospidin von 200 bis 240 µm Dicke und die zweite Schicht aus Äthylzyanakrylat von 20 bis 25 µm Dicke (0,I Teil von der Dicke der ersten Schicht) besteht. Der genannte Film wurde auf weiche Gewebe in der Zone der Geschwulstresektion appliziert. Die Prüfergebnisse im Vergleich zur Kontrolle (Injektionseinführung des Zytostatikums) sind in der nachfolgenden Tabelle angeführt.

Beispiel 2.

Ein geschwulsthemmender Film, dessen erste Schicht aus 90,16 Gew.% Kopolymer von N-Vinylpyrrolidon, Methylmethakrylat und Äthylalkrylat und 9,84 Gew.% Prospidin von 150 bis 200 µm Dicke und die zweite Schicht aus demselben Kopolymer von 120 bis 180 µm Dicke (0,8 Teile von der Dicke der ersten Schicht) besteht. Es wurden die Prüfungen ähnlich mit dem Beispiel I durchgeführt. Die Prüfergebnisse sind in der nachfolgenden Tabelle angeführt.

Beispiel 3.

Ein geschwulsthemmender Film, dessen erste Schicht aus 80 Gew.% Kopolymer von N-Vinylpyrrolidon und Butylmethakrylat und 20 Gew.% N'-Bis-(ß-chloräthyl)-N'-O-trimethylen-äther von 100 bis 120 µm Dicke und die zweite Schicht aus Polyglykolid von 50 bis 65 µm Dicke besteht. Es wurden die Prüfungen ähnlich mit dem Beispiel I durchgeführt. Die Prüfergebnisse sind in der nachfolgenden Tabelle angeführt.

Beispiel 4.

Ein geschwulsthemmender Film, dessen erste Schicht aus 70 Gew.% Kopolymer von N-Vinylpyrrolidon und Butylmethakrylat und 30 Gew.% Prospidin von 400 µm Dicke und die zweite Schicht aus demselben Kopolymer von 80 bis 100 µm Dicke besteht. Es wurden die Prüfungen ähnlich mit dem Beispiel I durchgeführt. Die Prüfergebnisse sind in der nachfolgenden Tabelle angeführt.

Beispiel 5.

Ein geschwulsthemmender Film, dessen erste Schicht aus 70 Gew.% Gemisch eines Kopolymers von N-Vinylpyrrolidon mit Butylmethakrylat und Polyäthoxyzyanakrylat (Gewichtsverhält-nis I:9) und 20 Gew.% Prospidin von 400 µm Dicke und die zweite Schicht aus Polyäthoxyzyanakrylat von 80 bis 100 µm Dicke besteht. Es wurden die Prüfungen ähnlich mit dem Beispiel I durchgeführt. Die Prüfergebnisse sind in der nachfolgenden Tabelle angeführt.

Beispiel 6.

Ein geschwulsthemmender Film, dessen erste Schicht aus dem Kopolymer von N-Vinylpyrrolidon und Butylmethakrylat und 20 Gew.% Prospidin von 400 µm Dicke und die zweite Schicht aus demselben Kopolymer von 80 bis 100 µm Dicke besteht. Die Prüfungen wurden ähnlich mit dem Beispiel I durchgeführt.

- 8 -

Die Prüfergebnisse sind in der nachfolgenden Tabelle angeführt.

Beispiel 7.

Ein geschwulsthemmender Film, dessen erste Schicht aus Polyäthylzyanakrylat und 20 Gew.% Prospidin von 400 $\mu$m Dicke, die zweite Schicht aus demselben Kopolymer von 80 bis 100 $\mu$m Dicke besteht. Die Prüfungen wurden ähnlich mit dem Beispiel I durchgeführt. Die Prüfergebnisse sind in der nachfolgenden Tabelle angeführt.

Tabelle I.

Prüfergebnisse des erfindungsgemäßen geschwulsthemmenden Films im Vergleich zur Kontrolle (Injektionseinführung des Zytostatikums)

| Lfd. Nr. | Erfindungsgemäßer geschwulsthemmender Film nach den angeführten Beispielen | Dicke der zweiten Filmschicht in $\mu$m und in Teilen von der Dicke der ersten Schicht | Zeit des Aufrechterhaltens der therapeutischen Konzentration in der Geschwulstzone bei der einmaligen Einführung in Tagen | |
|---|---|---|---|---|
| | | | Kontrolle | erfindungsgemäßer Film |
| I | 2 | 3 | 4 | 5 |
| I. | Nach dem Beispiel I | $\frac{20-25}{I}$ | 0,8-I,0 | I4-I6 |
| 2. | Nach dem Beispiel 2 | $\frac{I20-I60}{0,8}$ | 0,8-I,0 | 23-26 |
| 3. | Nach dem Beispiel 3 | $\frac{50-60}{0,5}$ | 0,8-I,0 | I0-I2 |
| 4. | Nach dem Beispiel 4 | $\frac{80-I00}{0,2-0,25}$ | 0,8-I,0 | 7-I0 |
| 5. | Nach dem Beispiel 5 | $\frac{80-I00}{0,2-0,25}$ | 0,8-I,0 | 8-II |
| 6. | Nach dem Beispiel 6 | $\frac{80-I00}{0,2-0,25}$ | 0,8-I,0 | 6-9 |
| 7. | Nach dem Beispiel 7 | $\frac{80-I00}{0,2-0,25}$ | 0,8-I,0 | 8-I0 |

Fortsetzung der Tabelle I

| Lfd. Nr. | Verlängerung der therapeutischen Einwirkungsdauer im Vergleich zur Kontrolle (um wieviel Mal) | Verbrauch am Zytostatikum | | Verminderung des Verbrauchs am Zytostatikum im erfindungsgemäßen Film (um wieviel Mal) |
| | | Kontrolle | erfindungsgemäßer Film | |
| I | 6 | 7 | 8 | 9 |
| 1. | 14-20 | 2-3,5 | 0,1 | 20-30 |
| 2. | 24-30 | 2,0-3,0 | 0,4 | 5-7 |
| 3. | 10-14 | 6-14 | 0,6-0,8 | 15-20 |
| 4. | 7-12 | 2-3 | 0,75 | 3-4 |
| 5. | 8-14 | 2-3 | 0,75 | 3-4 |
| 6. | 6-11 | 2-3 | 0,5 | 4-6 |
| 7. | 8-12 | 2-3 | 0,5 | 4-6 |

### Industrielle Anwendbarkeit

Der erfindungsgemäße geschwulsthemmende Film findet Verwendung in der Medizin zur Implantation (Applikation) in die Operationszone nach einer totalen oder subtotalen Exstirpation der Geschwulst von beliebiger Art, um eine langwierige lokale Einwirkung des aus dem Film langsam iliminierenden Arzneimittels auf die zurückgebliebenen Geschwulstzellen zu gewährleisten.

0268680

PATENTANSPRÜCHE:

I. Geschwulsthemmender Film aus biodestruktierbaren Polymeren, d a d u r o h g e k e n n z e i o h n e t, daß dieser einen zweischichtigen Film darstellt, in welchem die erste an die Geschwulstgewebe anliegende Schicht aus einem biodestruktierbaren Polymer und einem Zytostatikum und die zweite Schicht aus demselben oder einem anderen biodestruktierbaren Polymer besteht, das eine längere Biodestruktionsdauer als die Iliminierungsdauer des Zytostatikums aus der ersten Schicht hat, wobei die Dicke der zweiten Schicht O,I bis O,8 Teile von der Dicke der ersten Schicht beträgt.

2. Geschwulsthemmender Film nach Anspruch I, d a - d u r o h g e k e n n z e i o h n e t, daß dieser als biodestruktierbares Polymer der ersten Schicht ein Polymer auf Grundlage des Kopolymers aus N-Vinylpyrrolidon mit Alkylakrylat und/oder Alkylmethakrylat und/oder Zyanakrylat enthält.

3. Geschwulsthemmender Film nach Anspruch I bis 2, d a - d u r o h g e k e n n z e i o h n e t, daß dieser als biodestruktierbares Polymer der zweiten Schicht ein Polymer auf Grundlage von Polyglykolid, Karboxymethylzellulose oder ein Kopolymer aus N-Vinylpyrrolidon mit Alkylakrylat und/oder Alkylmethakrylat oder Zyanakrylat enthält.

International Application No PCT/SU 86/00052

## I. CLASSIFICATION OF SUBJECT MATTER (if several classification symbols apply, indicate all) *

According to International Patent Classification (IPC) or to both National Classification and IPC

IPC$^4$ :     A 61 L 15/03

## II. FIELDS SEARCHED

### Minimum Documentation Searched $^7$

| Classification System | Classification Symbols |
|---|---|
| IPC$^4$ : | A 61 L 15/00, 15/03, 15/04 |
| | A 61 K 31/765, 31/78, 31/785, 31/79 |

Documentation Searched other than Minimum Documentation
to the Extent that such Documents are Included in the Fields Searched *

## III. DOCUMENTS CONSIDERED TO BE RELEVANT *

| Category * | Citation of Document, $^{11}$ with indication, where appropriate, of the relevant passages $^{12}$ | Relevant to Claim No. $^{13}$ |
|---|---|---|
| A | WO, A1, 85/02343, (Vsesojuzny nauchno-issledovatelsky i ispytatelny institut meditsinskoi tekhniki) 6 June 1985, see the abstract | 1-3 |
| A | WO, A1, 86/00814, (KEY PHARMACEUTICALS, INC.) 13 February 1986 see the abstract | 1-3 |
| A | EP, A2, 0138740, (ENQUAY PHARMACEUTICAL ASSOCIATES) 24 April 1985 see the abstract | 1-3 |

* Special categories of cited documents: $^{10}$

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 5 January 1987 (05.01.87) | 26 February 1987 (26.02.87) |
| International Searching Authority | Signature of Authorized Officer |
| ISA/SU | |

Form PCT/ISA/210 (second sheet) (January 1985)